# EUROPEAN PATENT APPLICATION

(11) **EP 4 686 975 A1**
(43) Date of publication of application: **04.02.2026**
(21) Application number: 24191592.5
(22) Date of filing: 29.07.2024
(51) Int. Cl.: G02B 21/00, G06F 3/01, A61B 90/20

(54) **ROBOTIC MICROSCOPE SYSTEM, METHOD FOR CONTROLLING SUCH ROBOTIC MICROSCOPE SYSTEM AND COMPUTER PROGRAM PRODUCT**

(71) Applicant: BHS Technologies GmbH, 6020 Innsbruck (AT)
(72) Inventor: CAPELLI, Mark, 6020 Innsbruck (AT); SCHMID-SANTEK, Michael, 6091 Götzens (AT); KRAINER-ELLER, Philipp, 6020 Innsbruck (AT)
(74) Representative: Prüfer & Partner mbB Patentanwälte · Rechtsanwälte

(57) **Abstract**

The present invention relates to a robotic microscope system (100, 100') for imaging and displaying images of an operating field (6), comprising:
a microscopic imaging system (10, 10'), comprising at least one microscopic imaging unit (1), the at least one microscopic imaging unit (1) comprises at least one imaging device (8) providing a field of view (9) for imaging the operating field (6),
a robotic device (20), operatively connected to the microscopic imaging system (10, 10') configured for a controlled movement of the microscopic imaging system (10, 10') in at least one degree of freedom,
at least one display device (30) operatively connected to the at least one microscopic imaging unit (1) and configured to display at least the images of the operating field (6) imaged by the at least one imaging device (8),
at least a first detection device (3a) and a second detection device (3b), each of which providing a field of view (4a, 4b) for detecting at least one of a position, an orientation and a movement sequence of at least one control triggering object (5a, 5b) as at least one trigger characteristic, and
a control device (1b) configured to control at least one of the at least one microscopic imaging unit (1), the robotic device (20) and the display device (30) in accordance with the at least one detected trigger characteristic detected by at least one of the first detection device (3a) and the second detection device (3b) within a predetermined portion of the respective field of view (4a, 4b) for detecting the at least one trigger characteristic to adapt images of the operating field (6) to be displayed.

The present invention also relates to a method for controlling such robotic microscope system and a respective computer program product.

## Description

The present invention relates to a robotic microscope system for imaging and displaying images of an operating field, a method for controlling such robotic microscope system and a respective computer program product.

During microscopic surgical procedures, the imaging and/or display settings may have to be adapted to provide a desired view for a surgeon on a concrete task within an operating field. For example, a microscopic imaging unit may have to be positioned and/or oriented differently and/or a magnification has to be changed. A respective control may be provided by manual control members such as buttons or switches as manual input devices. However, a manual control causes sterility issues, specifically with the actual surgeon operating respective manual control members. Further, with respect to a surgeon operating manual control members, a manual control urges the surgeon the move her or his gaze to a position away from the operating field. Consequently, the attention of the surgeon to the surgical procedure may be temporarily interrupted.

For the avoidance of sterility issues and to allow the surgeon to maintain her or his attention on the operating field, a control of predetermined setting options may be executed by gesture control. For example, EP 3 285 107 A1 relates to a gesture control of a surgical microscope by detecting a movement of an object within a field of view of a detection unit. However, the limited field of view of the detection unit also limits the flexibility in control commands.

It is an object of the present invention to provide an improved control for a robotic microscope system, particularly with respect to flexibility.

The object is solved by the subject matter of the independent claims. Further aspects of the present invention are subject to the dependent claims.

According to the present invention a robotic microscope system for imaging and displaying images of an operating field comprises a microscopic imaging system, comprising at least one microscopic imaging unit, the at least one microscopic imaging unit comprises at least one imaging device providing a field of view for imaging the operating field. The robotic microscope system further comprises a robotic device, operatively connected to the microscopic imaging system configured for a controlled movement of the microscopic imaging system in at least one degree of freedom, at least one display device operatively connected to the at least one microscopic imaging unit and configured to display at least the images of the operating field imaged by the at least one imaging device. Additionally, the robotic microscope system comprises at least a first detection device and a second detection device, each of which providing a field of view for detecting at least one of a position, an orientation and a movement sequence of at least one control triggering object as at least one trigger characteristic, and a control device. The control device is configured to control at least one of the at least one microscopic imaging unit, the robotic device and the display device in accordance with the at least one detected trigger characteristic detected by at least one of the first detection device and the second detection device within a predetermined portion of the respective field of view for detecting the at least one trigger characteristic to adapt images of the operating field to be displayed.

In view of the above, the at least one control triggering object is an object to provide a gesture information to control the robotic microscope system accordingly. For example, the at least one control triggering object may be at least one of a hand, a finger, surgical instrument or the like. The gesture control is thereby not necessarily limited to a movement sequence of the at least one control triggering object but may alternatively or in addition consider a position and/or orientation of the at least one control triggering object.

The control device may thereby be configured to control the microscopic imaging unit, for example, to adapt optics such as a focal lens and/or a magnification optics, imaging settings and/or image processing settings. Alternatively or in addition, the control device may be configured to control the robotic device to change the position and/or orientation of the microscopic imaging system and the microscopic imaging unit, respectively. Further, alternatively or in addition, the control device may be configured to control the display device to adapt display settings such as display parameters and/or the position and/or orientation of the display, e.g. with respect to the surgeon.

To allow the control of the robotic microscope system as per the above, the robotic microscope system comprises at least two detection devices, i.e. at least the first detection device providing a first field of view and the second detection device providing a second field of view, wherein each of the first and second detection devices detecting the position, orientation and/or movement sequence of the at least one control triggering object within its respective field of view. Due to the at least two detection devices, the detection area may be enhanced, for example, to increase the space to execute gesture commands and/or allow better accessibility of the predetermined portion and thereby the flexibility in gesture control, and/or to provide some redundancy in the event of a malfunction of one of the detection devices or one of the fields of view of one of the detection devices being obstructed for detecting the control triggering object. The redundancy may not only relate to malfunctions but may also comprise redundancy to detect the control triggering object by different fields of view in accordance with an accessibility of a respective field of view or predetermined portion, respectively. For example, the predetermined portion of the field of view of the first detection device may be easier to be accessed by a left hand of a user, while the predetermined portion of the field of view of the second detection device may be easier accessed by a right hand of a user. Further, the at least two detection devices may allow a plausibility check on executed gestures.

The predetermined portion of the respective field of view is a portion of the field of view in which the position, orientation and/or movement sequence of the at least one control triggering object is detected to trigger a control command by the control device. In other words, even though the field of view of each of the first and second detection may allow detection of the position, orientation and/or movement sequence of the at least one control triggering object over its entire volume, triggering a control command by the control unit in response to a respective detection is restricted to the detection within the predetermined portion of the respective field of view. The predetermined portion may thereby be formed by the entire field of view or only portions thereof. The predetermined portion as only a portion of the field of view may be formed by a distinct section of the field of view covering a certain distance with respect to the respective detection device. For example, assuming the respective detection unit being positioned to provide a field of view extending vertically, the predetermined portion of the field of view for triggering command may extend in the vertical direction from a first distance from the respective detection device to a second distance from the respective detection device, wherein the first distance is less than the second distance.

The first and/or second detection device may comprise or be connected to a control unit or evaluation unit to allow to detect trigger characteristics of the control triggering object exclusively in the predetermined portion of the respective field of view and/or to transmit exclusively trigger characteristics detected in the predetermined portion of the respective field of view. The control unit or its respective functionality may be comprised by the control device, the respective detection device or a separate control unit. The control unit may be configured to provide the first and/or second detection device with respective settings for the respective field of view and/or the predetermined portion of the field of view. The evaluation unit may be configured to assign position data to detected trigger characteristics to sort for trigger characteristics within and without the predetermined portion of the respective field of view. The term "position data" is not restricted to concrete coordinates but may also comprise a position selection by image processing. For example, at least the first detection device may be an imaging device and the evaluation unit assigned to the first detection device may only transmit trigger characteristics recognized within a predetermined space representing the predetermined portion by image processing.

At least one of the fields of view and/or at least one of the predetermined portions of the field of view of the first and second detection devices may be adjustable. For example, the field of view of the first and/or second detection device may be adjusted according to a control command of the control device to change its size and/or orientation. Similarly, the control device may provide a control command to change the size, position and/or orientation of the predetermined portion of the field of view.

In some embodiments, at least one of the predetermined portion of the field of view of the first detection device and the predetermined portion of the field of view of the second detection device are separated from the field of view of the at least one imaging device.

Accordingly, the respective gesture control requires the gesture to be executed outside the field of view of the at least one imaging device for at least one of the detection devices. The view on the operating field may thereby not be obstructed by the execution of the gesture and the surgeon or another user can directly determine the effect of the control command in response to the gesture. Furthermore, the user has to intentionally move the control triggering object outside the field of view of the imaging device for the execution of a control command.

In some embodiments, at least one of the predetermined portion of the field of view of the first detection device and the predetermined portion of the field of view of the second detection device at least partially overlaps with the field of view of the at least one imaging device to form at least one image overlap portion.

Accordingly, for example, a control triggering object assumed to be controlled by a user viewing the image provided by the imaging device may be enabled to visually track the executed gesture. Further, assuming that the control trigger object may be associated with a hand of a user acting withing the field of view of the imaging device, the hand has not to be moved far away from the field of view of the imaging device and thereby a respective region of interest of the operating field. Consequently, the hand may be ready to be returned to the region of interest or operating field, respectively, without unnecessary delay. The imaging overlap portion may form the predetermined portion of the field of view of the respective detection device at least partially.

In some embodiments one of the predetermined portions of the field of view of the first or second detection device may be separated from the field of view of the imaging device, while the other predetermined portion of the field of view of the other detection device at least partially overlaps with the field of view of the at least one imaging device. In such configuration, the separated predetermined portion may be used to activate a control of at least one of the at least one microscopic imaging unit, the robotic device and the display device by the control device in accordance with a respective detected trigger characteristic. After such activation, the separated predetermined portion and/or at least the imaging overlap portion of the other detection device may be used for a subsequent control of the at least one microscopic imaging unit, the robotic device and the display device by the control device in accordance with detected trigger characteristics.

In some embodiments, the predetermined portion of the field of view of the first detection device and the predetermined portion of the field of view of the second detection device at least partially overlap to form a detection overlap portion.

The detection overlap portion may provide some redundancy with respect to a common predetermined portion of the predetermined portions of the field of view of the first and second detection device. Accordingly, for example, in the event of any malfunction of one of the first or the second detection device, the respective other detection device may still allow to detect trigger characteristics in the detection overlap portion.

Further, the detection overlap portion may also at least partially comprise the imaging overlap portion or may be separated from the imaging overlap portion, if any.

In some embodiments, the control device is configured to assign at least one control command in response to the at least one trigger characteristic detected in the predetermined portion of the field of view of the first detection device, the predetermined portion of the field of view of the second detection device, the at least one image overlap portion or the detection overlap portion differently from a control command assigned in response to the at least one trigger characteristic detected in at least another one of the predetermined portion of the field of view of the first detection device, the predetermined portion of the field of view of the second detection device, the at least one image overlap portion or the detection overlap portion.

Accordingly, alternatively or in addition to a redundancy with respect to the same commends assigned to the same trigger characteristics, at least one of the different portions is associated with a different control command being assigned to the same trigger characteristic than at least another portion. For example, a movement of the control triggering object in one direction, e.g. from left to right, in the predetermined portion of the field of view of the first detection device may trigger the control device to control the microscopic imaging unit to move in the same direction, i.e. from left to right as per the given example. However, the same movement of the control triggering object in the predetermined portion of the field of view of the second detection device may cause the control device to control the robotic device to move in the same direction or to control the imaging device to change a magnification. Further, with the predetermined portions of the field of view of the first and second detection device providing a detection overlap portion and/or at least one of the portions providing an imaging overlap portion, the robotic microscope system allows additional options to assign different control commands to the same trigger characteristic in accordance with the respective portion within the trigger characteristic is detected.

The assignment of different control commands to the same trigger characteristic in accordance with the respective portion within which the trigger characteristic is detected may be adaptable. For example, a respective assignment may differ depending on an operational mode of the robotic microscope system, a user input and/or other trigger events.

In some embodiments, the control device is configured to control at least one of the at least one microscopic imaging unit, the robotic device and the display device in accordance with the at least one combined trigger characteristic detected by at least one of the first detection device and the second detection device as a combination of at least one detected trigger characteristic of a first control triggering object and at least one detected trigger characteristic of a second control triggering object, preferably detected in the detection overlap portion.

For example, assuming the control triggering object as a left and a right hand of user, the at least one combined trigger characteristic may be defined as a particular trigger characteristic of the left hand and a particular trigger characteristic by the left hand. The respective particular trigger characteristics may be the same or different. Further, the respective particular trigger characteristics may have to be detected simultaneously or within a predetermined period of time.

The combined trigger characteristic may have to be detected within the predetermined portion of the field of view of the first and/or second detection device, within the detection overlap portion and/or within the imaging overlap portion.

The control device may be configured to change the assignment of control commands to single trigger characteristics to combined trigger characteristics in respective portions.

In some embodiments, at least one of the first detection device and the second detection device is configured to detect a distance.

The detection of a distance may allow to determine the trigger characteristic being executed within the predetermine portion with respect to the detected distance data. The first and/or second detection device may comprise one sensing unit to detect both, the trigger characteristic as well as a distance to an object such as the control triggering object and/or another object. Alternatively, the first and/or second detection device may comprise separated sensing units to detect the trigger characteristic as well as a distance to an object. By separate sensing units, the distance sensing unit may provide a smaller field of view than the trigger characteristic sensing unit to allow a more distinct distance detection. However, one sensing unit for both may be combined with a processing unit to assign different distances to different portions of the field of view of the sensing unit and to determine at least one of such portions as relevant for the distance detection. For example, the respective portion may be a fingertip or a tip of a surgical tool representing the distance to be detected.

In some embodiments, the control device is configured to control a focal length or working distance in accordance with the detected distance.

For example, the control device receives distance data with respect to a location a fingertip or tip of a surgical tool is pointing to. Here, the distance to be detected may be a maximum distance from the respective detection device in the direction of a focal distance adjacent to the fingertip or the tip of the surgical tool. Accordingly, the control device may be configured to adapt the optics for setting a focal plane in accordance with the detected maximum distance.

In some embodiments, the control device is configured to control the robotic device with respect to a collision prevention.

Accordingly, detecting a distance may allow the detection of potential collisions of any component of the robotic microscope system such as the imaging device with another object and/or the detection of potential collisions of the control triggering object approaching the robotic microscope system, e.g. the respective detection device, which may result in sterility issues. The distance to be detected for a collision prevention may be any distance falling below a predetermined threshold. With respect to a potential collision detection, the control device may initiate a warning signal, such as an acoustic and/or optic signal, and/or may be configured to control the robotic microscope system to prevent the collision, e.g. by moving the microscopic imaging unit in a more secure position or by stopping operation of any further movement.

In some embodiments, at least one of the first detection device and the second detection device is configured to detect the presence of a sterile cover.

The sterile cover may be an at least partially transparent film material or an at least partially transparent hard cover to at least partially cover the robotic microscope system. With the sterile cover being intended to also cover at least one of the first and second detection devices, the first and/or second detection device may allow to detect the presence of the sterile cover. For example, with respect to a distance detection, the respective detection device may detect the presence of the sterile cover with respect to a detected distance within a predetermined range. Alternatively or in addition, the presence of the sterile cover may generate an offset in the detected signals or any other change to signal data usually received without the presence of the sterile cover.

Further, the control device may be configured to stop or limit operation of the robotic microscope system if the sterile cover is not present and/or to trigger a respective warning, at least with respect to an operation mode associated with a surgical procedure requiring sterility.

In some embodiments, at least one of the first detection device and the second detection device comprises a radar-based detection member.

The radar-based detection member allows the detection of triggering characteristics as well as distances. Further, the radar-based detection member may ease the setting of predetermined portions of the field of view of the respective detection device. With respect to the detection of the presence of the sterile cover, the presence easily is detectable by a signal offset.

In some embodiments, the robotic microscope system comprises at least one manual control unit, wherein at least one of the first detection device and the second detection device is comprised by or attached to the at least one manual control unit.

The manual control unit may be attached to the microscopic imaging system, particularly to the at least one microscopic imaging unit. The manual control unit may be a handle or joystick to move the microscopic imaging system or microscopic imaging unit, respectively. Alternatively or in addition, the manual control unit may comprise at least an input device such as a touch screen, button, switch or the like to allow a user to control at least some functions of the robotic microscope system. With the first and/or second detection device being comprised by or attached to the manual control unit, the robotic microscope system may be easily retrofitted.

In some embodiments, the control device is configured to control at least one of the at least one microscopic imaging unit, the robotic device and the display device in accordance with at least one of the at least one control triggering object, the at least one detected trigger characteristic and a detection of a person associated with the at least one control triggering object representing a position of the person.

Accordingly, the control device or a respective processing unit is configured to determine or at least estimate a position of the person or user, respectively, by the at least one of the at least one control triggering object, the at least one detected trigger characteristic and a detection of the person. For example, with a left and a right hand of the person as control triggering objects, fingers of the left and right hand extending in substantially the same direction may be associated with the person being positioned in a direction along the arms opposite the fingertips. Alternatively or in addition, a trigger characteristic with the left hand and the right hand may require a specific position of the person to carry out the trigger characteristic in an ergonomic way. Alternatively or in addition, the person or at least a part of the person may be detectable by the first and/or second detection device. The position detection of the person or a part of the person may be carried out without activation of a respective control function by a predetermined trigger characteristic. Accordingly, the first and/or second detection device may provide the functionalities of executing control commands in accordance with a trigger characteristic and/or in accordance with the position detection. As another example, the detection of the person or at least a part of the person may be detectable by the first and/or second detection device only after a respective trigger characteristic has been determined to start the respective detection.

By determining or at least estimating the position of the person, the control device may control, for example, the microscopic imaging unit, the robotic device and/or the display device such that the displayed image corresponds to a view that allows a hand-eye-coordinated operation by the person. Further, the robotic device may be controlled to avoid any arm of the robotic device being moved into an area associated with the position of the person.

In a further aspect, the present invention relates to a method for controlling a robotic microscope system as previously described, comprising the steps of:
- detecting at least one of a position, an orientation and a movement sequence of at least one control triggering object as at least one trigger characteristic, and
- controlling at least one of at least one microscopic imaging unit, a robotic device and a display device of the robotic microscope system in accordance with the at least one detected trigger characteristic.

Any feature of the robotic microscope system may also apply for the method with respect to a described functionality. Vice versa, any feature of the method with respect to the robotic microscope system may also apply for the robotic microscope system.

In a further aspect, the present invention relates to a computer program product comprising instructions which, when the program is executed by a computer, cause the computer to carry out the method as previously described.

Accordingly, any method feature may also apply to the computer program product.

Further advantages, aspects and details of the invention are subject to the claims, the following description of exemplary embodiments applying the principles of the invention, and the respective exemplary drawings.
Figure 1 is a schematic illustration of a robotic microscope system according to a first exemplary embodiment; and
Figure 2 is a schematic illustration of a robotic microscope system according to a second exemplary embodiment.

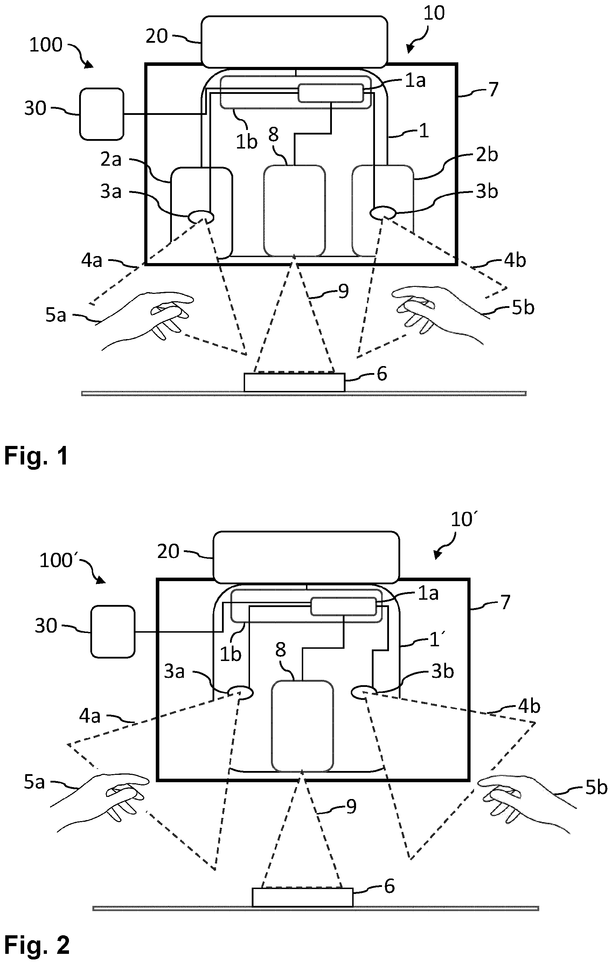

Figure 1 shows a schematic illustration of a robotic microscope system 100 according to a first exemplary embodiment. The robotic microscope system 100 comprises a microscopic imaging system 10 and a robotic device 20 capable of moving the microscopic imaging device 10 in six degrees of freedom. However, in alternative embodiments, the degrees of freedom provided by the robotic system may deviate from six.

The microscopic imaging system 10 comprises a microscopic imaging unit 1 with an imaging device 8 having a field of view 9 to image an operating field 6. The imaging device 8 may be a stereoscopic camera or a 3D camera. The image is transferred to a display device 30, here a head-mounted display, via a processing unit 1a comprised by a control device 1b of the microscopic imaging unit 1. However, in alternative embodiments, the processing unit 1a may be separate from the control device 1b, and the processing unit 1a and the control device 1b may be separate from the microscopic imaging unit 1 and the microscopic imaging system 10. Further, in other embodiments, the display device may be an external monitor such as an external 3D monitor.

The control device 1b is configured to control the microscopic imaging system 10, the robotic device 20 and the display device 30. In the exemplary embodiment, the control device 1b is configured to control the movement of the robotic device 20, the imaging settings of the imaging device 8, and the display settings of the display device 30.

The above control by the control device 1b can be executed by gesture control.

Accordingly, the microscopic imaging system 10 comprises a first detection device 3a and a second detection device 3b. The first detection device 3a is comprised by a first manual control unit 2a, and the second detection device 3b is comprised by a second manual control unit 2b. The first and second manual control units 2a, 2b are arranged on opposite sides of the imaging device 8. The opposite arrangement relates to lateral sides of the imaging device 8 with respect to field of view 9 of the imaging device 8.

Each of the first detection device 3a and the second detection device 3b provides a field of view 4a, 4b. In the exemplary embodiment, the field of view 4a of the first detection device 3a corresponds to a predetermined portion of the field of view 4a to detect a trigger characteristic for the control device 1b initiating a respective control.

Similar, the field of view 4b of the second detection device 3b corresponds to a predetermined portion of the field of view 4b to detect a trigger characteristic for the control device 1b initiating a respective control. However, in alternative embodiments, the predetermined portion of the field of view 4a of the first detection device 3a and/or the predetermined portion of the field of view 4b of the second detection device 3b may only be a section of the field of view 4a of the first detection device 3a or the field of view 4b of the second detection device 3b, respectively.

The field of view 4a of the first detection device 3a and the field of view 4b of the second detection device 3b do not overlap with each other or with field of view 9 of the imaging device 8. Accordingly, each of the first detection device 3a and the second detection device 3b provides an independent field of view 4a, 4b. However, in other embodiments, the field of view 4a of the first detection device 3a and the field of view 4b of the second detection device 3b may at least partially overlap with each other to provide a detection overlap portion, and/or at least one of the field of view 4a of the first detection device 3a and the field of view 4b of the second detection device 3b may at least partially overlap to provide an image overlap portion.

For the gesture control, control triggering objects 5a, 5b are to be introduced into the respective field of view 4a, 4b. In the exemplary embodiment, the control triggering object 5a is represented by a left hand, and the other control triggering object 5b is represented by a right hand. The control device 1b is configured to assign different control commands with respect to the microscopic imaging system 10 and the robotic device 20 to the same gesture depending on whether the gesture as triggering characteristic is detected in the field of view 4a of the first detection device 3a or in the field of view 4b of the second detection device 3b. Here, the control commands assigned to the same triggering characteristic in the field of view 4a of the first detection device 3a relates to the microscopic imaging system 10, while the control commands assigned to the same triggering characteristic in the field of view 4b of the second detection device 3b relates to the robotic system. Other trigger characteristics relating to the control of the display device 30 have the same assignment of control commands independent of the respective trigger characteristic being detected in the field of view 4a of the first detection device 3a or in the field of view 4b of the second detection device 3b. Accordingly, the microscopic imaging system 10 can be easily controlled by the left hand as the control triggering object 5a, the robotic device 20 can be easily controlled by the right hand as the control triggering object 5b, and the display device can be easily controlled by each of the left and right hand as control triggering objects 5a, 5b with a limited number of triggering characteristics to be learned by the user as well as the control device 1b.

In the exemplary embodiment, each of the first and second detection devices 3a, 3b comprises a radar-based detection member for detecting the respective trigger characteristics. The radar-based detection members are also implemented to detect the presence of a sterile cover 7 covering the microscopic imaging system 10. If the sterile cover 7 is present, the radar signal data shows an offset indicative of the presence of the sterile cover 7. This offset is recognized by the processing unit 1a and received by the control device 1a to initiate a warning.

Figure 2 shows a schematic illustration of a robotic microscope system 100' according to a second exemplary embodiment. The second embodiment differs from the first embodiment by the positioning of the first detection device 3a and the second detection device 2a. Accordingly, only respective differences of the second embodiment with respect to the first embodiment are described and reference signs for the first embodiment also apply for the second embodiment, wherein components affected by the difference are indicated by an apostrophe.

In the second embodiment, the first detection device 3a and the second detection device 3b are comprised by the microscopic imaging unit 1' or microscopic imaging system 10', respectively. This may allow the first detection device 3a and the second detection device 3b to be positioned independent of any manual control units.

The invention has been described with respect to exemplary embodiments. However, the invention is not limited to the exemplary embodiments. For example, the robotic microscope system may further comprise a foot switch and/or another input device to activate the control by detected trigger characteristics. The input device such as the foot switch may also be used for the release of other control functions.

### List of reference signs

- 1: microscopic imaging unit
- 1a: processing unit
- 1b: control device
- 2a: first manual control unit
- 2b: second manual control unit
- 3a: first detection device
- 3b: second detection device
- 4a, 4b: field of view (detection device)
- 5a, 5b: control triggering object
- 6: operating field
- 7: sterile cover
- 8: imaging device
- 9: field of view (imaging device)
- 10, 10': microscopic imaging system
- 20: robotic device
- 30: display device
- 100, 100': robotic microscope system

## Claims

1. Robotic microscope system (100, 100') for imaging and displaying images of an operating field (6), comprising:
a microscopic imaging system (10, 10'), comprising at least one microscopic imaging unit (1), the at least one microscopic imaging unit (1) comprises at least one imaging device (8) providing a field of view (9) for imaging the operating field (6),
a robotic device (20), operatively connected to the microscopic imaging system (10, 10') configured for a controlled movement of the microscopic imaging system (10, 10') in at least one degree of freedom,
at least one display device (30) operatively connected to the at least one microscopic imaging unit (1) and configured to display at least the images of the operating field (6) imaged by the at least one imaging device (8),
at least a first detection device (3a) and a second detection device (3b), each of which providing a field of view (4a, 4b) for detecting at least one of a position, an orientation and a movement sequence of at least one control triggering object (5a, 5b) as at least one trigger characteristic, and
a control device (1b) configured to control at least one of the at least one microscopic imaging unit (1), the robotic device (20) and the display device (30) in accordance with the at least one detected trigger characteristic detected by at least one of the first detection device (3a) and the second detection device (3b) within a predetermined portion of the respective field of view (4a, 4b) for detecting the at least one trigger characteristic to adapt images of the operating field (6) to be displayed.

2. The robotic microscope system (100, 100') according to claim 1, wherein at least one of the predetermined portion of the field of view (4a) of the first detection device (3a) and the predetermined portion of the field of view (4b) of the second detection device (3b) are separated from the field of view (9) of the at least one imaging device (8).

3. The robotic microscope system (100, 100') according to claim 1 or 2, wherein at least one of the predetermined portion of the field of view (4a) of the first detection device (3a) and the predetermined portion of the field of view (4b) of the second detection device (3b) at least partially overlaps with the field of view (9) of the at least one imaging device (8) to form at least one image overlap portion.

4. The robotic microscope system (100, 100') according to any one of the preceding claims, wherein the predetermined portion of the field of view (4a) of the first detection device (3a) and the predetermined portion of the field of view (4b) of the second detection device (3b) at least partially overlap to form a detection overlap portion.

5. The robotic microscope system (100, 100') according to any one of the preceding claims, wherein the control device (1b) is configured to assign at least one control command in response to the at least one trigger characteristic detected in the predetermined portion of the field of view (4a) of the first detection device (3a), the predetermined portion of the field of view (4b) of the second detection device (3b), the at least one image overlap portion or the detection overlap portion differently from a control command assigned in response to the at least one trigger characteristic detected in at least another one of the predetermined portion of the field of view (4a) of the first detection device (3a), the predetermined portion of the field of view (4b) of the second detection device (3b), the at least one image overlap portion or the detection overlap portion.

6. The robotic microscope system (100, 100') according to any one of the preceding claims, wherein the control device (1b) is configured to control at least one of the at least one microscopic imaging unit (1), the robotic device (20) and the display device (30) in accordance with the at least one combined trigger characteristic detected by at least one of the first detection device (3a) and the second detection device (3b) as a combination of at least one detected trigger characteristic of a first control triggering object (5a) and at least one detected trigger characteristic of a second control triggering object (5b), preferably detected in the detection overlap portion.

7. The robotic microscope system (100, 100') according to any one of the preceding claims, wherein at least one of the first detection device (3a) and the second detection device (3b) is configured to detect a distance.

8. The robotic microscope system (100, 100') according to claim 7, wherein the control device (1b) is configured to control a focal length or working distance in accordance with the detected distance.

9. The robotic microscope system (100, 100') according to claim 7 or 8, wherein the control device (1b) is configured to control the robotic device (20) with respect to a collision prevention.

10. The robotic microscope system (100, 100') according to any one of the preceding claims, wherein at least one of the first detection device (3a) and the second detection device (3b) is configured to detect the presence of a sterile cover (7).

11. The robotic microscope system (100, 100') according to any one of the preceding claims, wherein at least one of the first detection device (3a) and the second detection device (3b) comprises a radar-based detection member.

12. The robotic microscope system (100) according to any one of the preceding claims, wherein the robotic microscope system (100) comprises at least one manual control unit (2a, 2b), wherein at least one of the first detection device (3a) and the second detection device (3b) is comprised by or attached to the at least one manual control unit (2a, 2b).

13. The robotic microscope system (100, 100') according to any one of the preceding claims, wherein the control device (1b) is configured to control at least one of the at least one microscopic imaging unit (1), the robotic device (20) and the display device (30) in accordance with at least one of the at least one control triggering object (5a, 5b), the at least one detected trigger characteristic and a detection of a person associated with the at least one control triggering object (5a, 5b) representing a position of the person.

14. Method for controlling a robotic microscope system (100, 100') according to any one of the claims 1 to 13, comprising the steps of
- detecting at least one of a position, an orientation and a movement sequence of at least one control triggering object (5a, 5b) as at least one trigger characteristic, and
- controlling at least one of at least one microscopic imaging unit (1), a robotic device (20) and a display device (30) of the robotic microscope system (100, 100') in accordance with the at least one detected trigger characteristic.

15. Computer program product comprising instructions which, when the program is executed by a computer, cause the computer to carry out the method of claim 14.
